(19) Europäisches Patentamt

European Patent Office

Office européen des brevets

(11) **EP 0 987 917 B1**

(12) **FASCICULE DE BREVET EUROPEEN**

(45) Date de publication et mention
de la délivrance du brevet:
**02.11.2006 Bulletin 2006/44**

(51) Int Cl.:
***H04Q 11/04*** *(2006.01)*

(21) Numéro de dépôt: **98460038.7**

(22) Date de dépôt: **18.09.1998**

(54) **Procédé de transmission de données organisées en cellules, cellule, système de communication et unité terminale de réseau correspondante**

Verfahren zur zellularen Datenübertragung, Zelle, Kommunikationssystem und entsprechendes Netz-Endgerät

Method of cell data transmission, cell, communication system and corresponding network terminal unit

(84) Etats contractants désignés:
**DE FR GB**

(43) Date de publication de la demande:
**22.03.2000 Bulletin 2000/12**

(73) Titulaire: **NEWBRIDGE NETWORKS CORPORATION**
**Kanata, Ontario K2K 2E6 (CA)**

(72) Inventeurs:
- **Normand, Dominique**
  **35170 Bruz (FR)**
- **André, Marc**
  **35510 Cesson Sevigne (FR)**
- **Malorey, Didier**
  **35250 Chevaigné (FR)**
- **Ambrosio, Esabel**
  **35135 Chantepie (FR)**

(74) Mandataire: **Vidon, Patrice**
**Cabinet Vidon**
**16 B, rue Jouanet - B.P. 90333**
**Technopole Atalante**
**35703 Rennes Cedex 7 (FR)**

(56) Documents cités:
**US-A- 5 745 837**

- **RAYCHAUDHURI D: "ATM BASED TRANSPORT ARCHITECTURE FOR MULTISERVICES WIRELESS PERSONAL COMMUNICATION NETWORKS" SERVING HUMANITY THROUGH COMMUNICATIONS. SUPERCOMM/ICC, NEW ORLEANS, MAY 1 - 5, 1994, vol. 1, 1 mai 1994, pages 559-565, XP000438976 INSTITUTE OF ELECTRICAL AND ELECTRONICS ENGINEERS**

**Description**

Le domaine de l'invention est celui des systèmes de communication asynchrone, assurant la transmission de données entre deux ou plusieurs éléments de réseau, sous la forme d'unités de données de protocole (PDU) de taille (s) fixe(s). Notamment, mais non exclusivement, l'invention concerne les systèmes de communication mettant en oeuvre des cellules au format ATM.

La technique ATM ("Asynchronous Transfer Mode" ou "Mode de Transfert Asynchrone") est bien connue. D'une façon générale, les données transitent à haut débit, via un réseau, sous la forme de cellules. Chaque cellule comprend un champ en-tête et un champ d'information (ou charge utile). Le champ en-tête permet le multiplexage (ou routage) de la cellule associée, à travers les différents éléments du réseau (terminaux, multiplexeurs, brasseurs, commutateurs...). Le champ d'informations comporte des données utiles destinées à un usager du réseau, destinataire de la cellule.

Ces deux champs présentent un format fixe: le champ en-tête comprend 5 octets de données et le champ d'informations comprend 48 octets de données. Parmi les 5 octets de données du champ en-tête, on distingue deux champs permettant d'identifier le multiplexage, à savoir 8 bits pour un champ d'identification de chemin virtuel complet (ou VPI, pour "Virtual Path Identifier" en anglais) et 16 bits pour un champ d'identification de canal virtuel (ou VCI, pour "Virtual Channel Identifier" en anglais).

En général, on dispose de moyens de transmission très haut débit, dans un réseau ATM. C'est notamment le cas entre des équipements intermédiaires constitutifs du réseau. En revanche, des problèmes sont souvent rencontrés "en bout de ligne", pour assurer une distribution jusqu'aux usagers à des débits suffisants.

En effet, la liaison entre le réseau et l'abonné final est souvent assurée sur une ligne téléphonique, qui supporte difficilement un débit de plusieurs Mbits/s.

Sur cette liaison d'extrémité, on peut mettre en oeuvre une modulation DSL (ou une modulation dérivée, du type ADSL, HDSL, SDSL,...) qui peut fonctionner jusqu'à plusieurs Mbits/s.

Toutefois, cela peut être insuffisant pour supporter tous les services. Il est à noter qu'outre la partie principale de la bande passante utilisée pour véhiculer les informations utiles du champ d'informations, une partie supplémentaire de la bande passante est utilisée pour transporter le champ en-tête. De plus, une partie supplémentaire de la bande passante doit également être réservée à l'administration des équipements (destinataires des cellules).

Dans de telles conditions, une optimisation de la bande passante est, par conséquent, souhaitable.

McTiffin a proposé une solution à ce problème, dans le cas particulier d'une transmission hertzienne entre le réseau ATM et l'abonné. Cette technique, décrite dans le document de brevet US 5,406,550, propose de supprimer les champs d'identification VPI et VCI et d'utiliser un codage radio identifiant le destinataire.

Cependant, cette solution présente de nombreux inconvénients.

Tout d'abord, une telle solution est dédiée uniquement aux transmissions à liaison radio pour lesquelles il est nécessaire que le signal émis porte une identification (le codage radio) du destinataire. Cette identification n'existe pas dans le cas des réseaux filaires. Ajouter une telle information nécessiterait une augmentation de la capacité de traitement de l'équipement terminal d'extrémité (NTU), de façon inutile, puisque le lien entre NTU et le réseau est unique.

Plus précisément encore, la technique de McTiffin est réservée à des techniques d'Accès Multiple à Répartition par Codes (ou "CDMA" pour "Coded Division Multiple Access" en anglais) ou d'Accès Multiple à Répartition Temporelle (ou "TDMA" pour "Time Division Multiple Access" en anglais), qui dispose de moyens de découpage de cellule permettant d'encapsuler un nombre entier de cellules ATM à l'intérieur d'une trame radio. Or, une telle encapsulation nécessite un supplément de bande passante, pour l'introduction d'un silence entre deux cellules destinées à un même destinataire.

Cette solution est donc compatible uniquement avec un système de transmission radio qui délimite les cellules pour les séparer et exige donc des moyens de transmission spécifiques adaptés.

Enfin, cette solution n'est pas compatible avec une transmission d'un flux continu de cellules, la synchronisation étant assurée par le réseau de transmission.

On trouve dans l'art antérieur des techniques d'optimisation de la bande passante, particulièrement dans le cas de réseau ATM.

De telles techniques sont présentées par exemple dans l'article IEEE « ATM Based trasport architecture for Multiservices Wireless Personal Communication Networks » du 15 mai 1994, ou dans le document de brevet n° 5,745,837.

La présente invention a notamment pour objectif de pallier ces différents inconvénients de l'état de la technique.

Plus précisément, l'un des objectifs de la présente invention est de fournir un *procédé de transmission de données organisées en cellules* (en particulier de type ATM) permettant d'obtenir un gain en bande passante sur une portion de la transmission, et notamment aux extrémités d'un réseau de transmission.

Un objectif complémentaire de l'invention est de fournir un tel procédé permettant d'assurer des services supplémentaires par rapport à la simple transmission de cellules, notamment sur une liaison terminale de type téléphonique classique (par exemple services concernant la location, le relais de trames "frame relay", Internet, ...).

Un autre objectif de l'invention est de fournir un tel procédé qui soit indépendant du système de transmission mis en oeuvre au niveau de l'abonné. Notamment, l'invention a pour objectif de fournir un tel procédé, fonctionnant sur tout type de réseau (hertzien, filaire,...).

Un autre objectif de l'invention est de fournir un tel procédé permettant de simplifier, ou à tout le moins de ne pas complexifier l'administration du réseau.

L'invention a également pour objectif de fournir un système de communication ainsi qu'une unité terminale de réseau d'un tel système, mettant en oeuvre un tel procédé.

Ces objectifs, ainsi que d'autres qui apparaîtront par la suite, sont atteints selon l'invention à l'aide d'un procédé de transmission de données organisées en cellules, comprenant chacune un en-tête dit en-tête complet et une zone de données, comprenant une étape de réduction de la taille de chacun d'au moins certains desdits en-têtes, entre deux équipements de communication d'une chaîne de transmission, pour former des en-têtes réduits par suppression d'éléments de données prédéterminés dans lesdits en-têtes complets, au moins certains desdits équipements comprenant des moyens de reconstruction desdits en-têtes complets, à partir desdits en-têtes réduits.

Ainsi, l'invention repose sur une approche nouvelle de la transmission des cellules. En effet, au lieu de proposer l'utilisation de moyens de transmission plus performants de cellules de taille fixe, on propose la mise en oeuvre de moyens de compression de cellules au sein de l'équipement de communication d'émission des cellules, et d'autre part des moyens de décompression des cellules comprimées au sein de l'équipement de communication de réception des cellules. Il n'est ainsi pas utile d'augmenter la bande passante nécessaire.

Le principe général de l'invention repose donc sur le compactage de certains champs des en-têtes des cellules transmises, par une suppression d'éléments de données ' non utilisés sur la portion de transmission considérée.

Il est donc possible d'augmenter le nombre de cellules, et donc le nombre de données utiles, à débit constant dans le réseau de transmission. En d'autres termes, on augmente la bande passante exploitable au sein des équipements de communication, notamment ceux situés aux extrémités de ligne.

On notera que le procédé selon l'invention de transmission de données organisées en cellules n'est pas obligatoirement symétrique. Ce procédé peut être mis en oeuvre dans un seul sens de transmission.

De façon avantageuse, ladite étape de réduction de la taille des en-têtes est mise en oeuvre sur une ligne d'extrémité, alimentant une unité terminale de réseau (ou "Network Termination Unit" en anglais). Cependant, le cas échéant, d'autres "zones de transmission" peuvent mettre en oeuvre cette technique.

Selon un mode de réalisation préférentiel de l'invention, ledit en-tête réduit comprend un identifiant de chemin virtuel réduit codé sur 4 bits, correspondant aux 4 bits de poids faible dudit identifiant de chemin virtuel complet ("VPI" en anglais), et un identifiant de canal virtuel réduit codé sur 8 bits, correspondant aux 8 bits de poids faible dudit identifiant de canal virtuel complet ("VCI"), lesdits équipements reconstruisant lesdits identifiants (VPI et VCI) complets par insertion de 4 et 8 zéros respectivement pour former les bits de poids fort.

On obtient ainsi une réduction de 12 bits sur la taille de chaque cellule.

Avantageusement, le champ de contrôle de flux générique (ou "GFC" pour "Generic Flow Control" en anglais) est supprimé dans ledit en-tête réduit, lesdits équipements reconstruisant le champ de contrôle de flux générique par insertion de 4 zéros.

Selon un mode de réalisation préférentiel de l'invention, ledit en-tête réduit et ledit en-tête complet comprennent un champ de détection et de correction d'erreur (ou "HEC" pour "Header Error Control" en anglais) codé sur 8 bits, l'algorithme de détection et de correction ne portant que sur les bits formant ledit en-tête réduit.

Selon une variante avantageuse, ledit en-tête réduit comprend un champ de détection et de correction d'erreur réduit, de moins de 8 bits, l'algorithme de détection et de correction ne portant que sur les bits formant ledit en-tête réduit. Ledit champ de détection et de correction réduit comprend par exemple 5 bits et est calculé à l'aide d'un code de Hamming.

Selon un premier mode de réalisation de l'invention, ladite étape de réduction est mise en oeuvre, de façon systématique, entre deux équipements prédéterminés.

Les équipements prédéterminés peuvent notamment être, d'une part, un équipement placé à l'origine d'une ligne d'une chaîne de transmission, et d'autre part un équipement placé à l'extrémité de cette ligne de la chaîne de transmission.

Selon un second mode de réalisation de l'invention, un premier desdits équipements décide, lors de l'établissement d'une liaison et/ou en cours de transmission, de la mise en oeuvre de ladite étape de réduction et/ou des conditions de mise en oeuvre correspondantes, le second desdits équipements adoptant ces décisions.

Selon un troisième mode de réalisation de l'invention, la mise en oeuvre de ladite étape de réduction et/ou des conditions de mise en oeuvre de ladite étape de réduction est négociée entre lesdits équipements en cours de transmission.

Avantageusement, lesdites conditions de mise en oeuvre comprennent la taille d'au moins un champ dudit en-tête réduit.

De façon avantageuse, ledit procédé comprend également une étape de synchronisation, dans chacun desdits équipements, consistant à rechercher le début de cellules complètes et/ou de cellules réduites.

Cette étape de synchronisation peut permettre la détermination de la décision dudit premier équipement.

En d'autres termes, l'étape de synchronisation permet à un équipement de communication intermédiaire et/ou récepteur des cellules transmises de déterminer le(s) choix du ou des format(s) de réduction opéré sur les cellules réduites par l'équipement de communication émetteur de telles cellules.

L'invention concerne également les cellules réduites en tant que telles, créées selon le procédé décrit précédemment. Elles comprennent chacune un en-tête et une zone de données, selon laquelle, ledit en-tête comprend un nombre réduit d'éléments de données, certains des éléments de données dudit en-tête étant supprimés, et ladite cellule est transmise entre deux équipements de communication d'une chaîne de transmission, ces derniers comprenant des moyens de reconstruction desdits éléments de données supprimés.

L'invention concerne encore un système de communication mettant en oeuvre le procédé décrit ci-dessus, dans lequel au moins deux desdits équipements échangent au moins certaines cellules comprenant un en-tête réduit, obtenu par suppression d'éléments de données dudit en-tête complet, non utilisés entre lesdits équipements, lesdits équipements comprenant des moyens de reconstruction dudit en-tête complet, à partir dudit en-tête réduit.

L'invention concerne aussi les unités terminales de réseau mises en oeuvre dans le système décrit ci-dessus, et comprenant des moyens de réduction de la taille d'un en-tête complet, pour former un en-tête réduit et des moyens de reconstruction d'un en-tête complet, à partir d'un en-tête réduit

D'autres caractéristiques et avantages de l'invention apparaîtront à la lecture de la description suivante d'un mode de réalisation préférentiel de l'invention, donné à titre de simple exemple illustratif et non limitatif, et en référence aux dessins annexés, dans lesquels :

- la figure 1 présente un organigramme simplifié d'un mode de réalisation particulier d'un procédé de transmission de données organisées en cellules, selon l'invention;
- la figure 2 présente un schéma simplifié d'un mode de réalisation particulier d'une cellule réduite obtenue par la mise en oeuvre du procédé de la figure 1 en comparaison avec une cellule complète ;
- la figure 3 présente un schéma simplifié d'un mode de réalisation particulier d'un réseau d'un système de transmission pouvant mettre en oeuvre le procédé de la figure 1 ; et
- la figure 4 présente un schéma simplifié d'un mode de réalisation particulier d'un ensemble fonctionnel d'adaptation au procédé de la figure 1.

La présente invention concerne donc un procédé de transmission de données organisées en cellules, permettant une transmission dans de bonnes conditions même lorsque le débit est limité, notamment aux extrémités du réseau.

Dans la suite de la présente description, on considère, à titre d'exemple, un réseau d'un système de transmission de données organisées en cellules selon une technique de transfert asynchrone standard ATM.

De façon classique, une cellule comprend un en-tête, permettant de désigner l'adresse de son destinataire, et une zone de données, véhiculant les données utiles destinées au récepteur de cette cellule.

Selon l'invention, le procédé comprend une étape de réduction de la taille d'au moins certains des en-têtes, entre deux équipements de communication d'une chaîne de transmission, pour former des en-têtes réduits par suppression d'éléments de données prédéterminés dans les en-têtes complets, au moins un des équipements comprenant des moyens de reconstruction des en-têtes complets, à partir des en-têtes réduits.

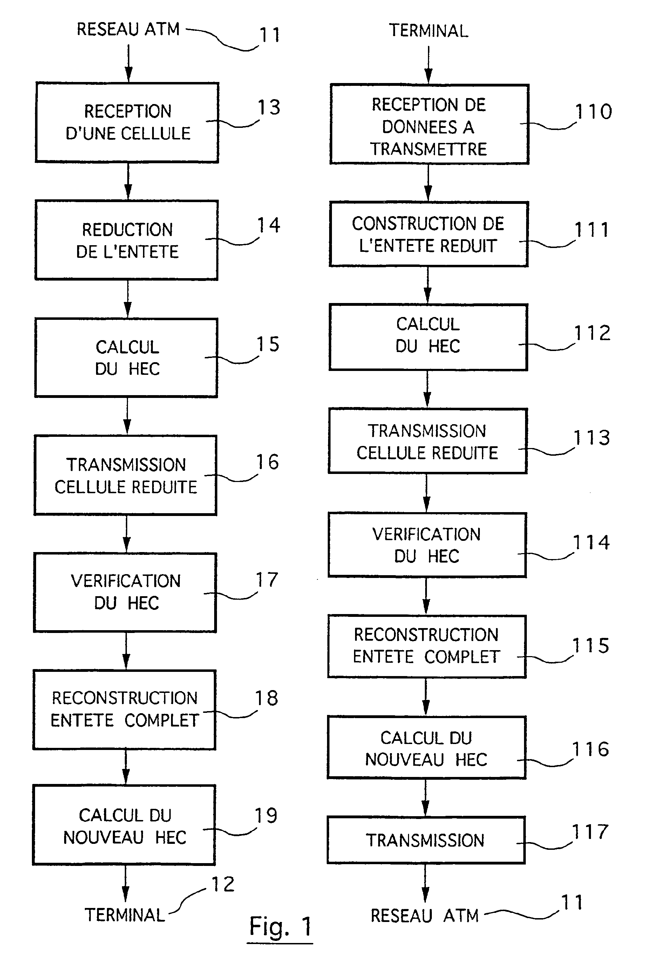

On présente maintenant, en relation avec l'organigramme de la figure 1, un mode de réalisation particulier du procédé selon l'invention de transmission de données organisées en cellules.

Ce mode de réalisation particulier concerne la transmission des cellules ATM entre un réseau ATM et un terminal d'usager, en utilisant une ligne de transmission classique. Comme déjà indiqué, le procédé de l'invention peut être mis en oeuvre sélectivement dans un seul sens de transmission. On a représenté sur la figure 1 indépendamment, ces deux sens de transmission.

Dans le sens réseau ATM 11 vers terminal 12, lorsqu'on reçoit 13 une cellule à faire parvenir au terminal 12, on procède à la réduction 14 de son en-tête. Cette opération repose sur la suppression d'informations de l'en-tête de la cellule, qui ne sont pas utilisées pour la transmission entre le réseau et le terminal. Ainsi, on réduit la taille des en-têtes et donc des cellules. A débit constant, il est alors possible de transmettre un plus grand nombre de cellules réduites.

En d'autres termes, le procédé de l'invention crée de la place disponible pour d'autres cellules. Cette place est intéressante notamment pour des lignes d'accès à des réseaux de systèmes de transmission fonctionnant à des vitesses de transmission de cellules relativement basses, c'est-à-dire d'environ quelques kbits/s à environ quelques ' Mbits/s. En effet, un gain de bande passante (exprimé classiquement en termes de débit, c'est-à-dire en nombre de cellule(s) par unité de temps), même de l'ordre d'un petit pourcentage, peut être souhaité pour accéder à des données utiles supplémentaires. Cet accès à des données supplémentaires permet une meilleure exploitation des fonctionnalités disponibles chez l'usager. L'usager final peut ainsi disposer de services supplémentaires.

Avantageusement, selon l'invention, le calcul de données de détection et/ou de correction d'erreurs (HEC) est effectué (15) sur les données de l'en-tête réduit Le champ correspondant peut également être réduit. Selon un autre mode de réalisation, il est conservé à sa taille initiale, et permet une meilleure correction d'erreurs, du fait qu'il est calculé sur un nombre réduit de bits.

La cellule réduite est ensuite transmise (16) de l'extrémité du réseau ATM 11 vers le terminal 12. On effectue (17) une vérification du champ de détection et de correction d'erreurs HEC, à partir du HEC calculé à l'étape 15. Dans l'unité terminale de réseau (NTU), on reconstruit (18) la cellule complète, en y recréant les données qui ont été supprimées. Cette étape n'est cependant pas obligatoire, puisque la cellule est arrivée à destination.

On calcule (19) enfin le nouveau champ de détection et de correction d'erreurs HEC associé à la cellule complète, et on délivre les données obtenues au terminal 12.

Dans l'autre sens de transmission, du terminal 12 au réseau ATM 11, on construit (111) directement une cellule à en-tête réduit, à partir de données à transmettre (110). On calcule (112) des données de détection et de correction d'erreurs sur cet en-tête réduit

Puis, on transmet (113) la cellule réduite vers l'extrémité du réseau ATM. Dans l'équipement du réseau ATM correspondant, on effectue (114) une vérification du champ de détection et de correction d'erreurs HEC, à partir du HEC établi en l'étape 112.

Ensuite, on reconstruit (115) la cellule complète, en y rétablissant les données qui ont été supprimées au sein de l'en-tête.

La cellule étant reconstruite, on calcule (116) le nouveau champ de détection et de correction d'erreurs HEC associé à la cellule complète.

La cellule complète est alors, classiquement, transmise (117) sur le réseau ATM 11.

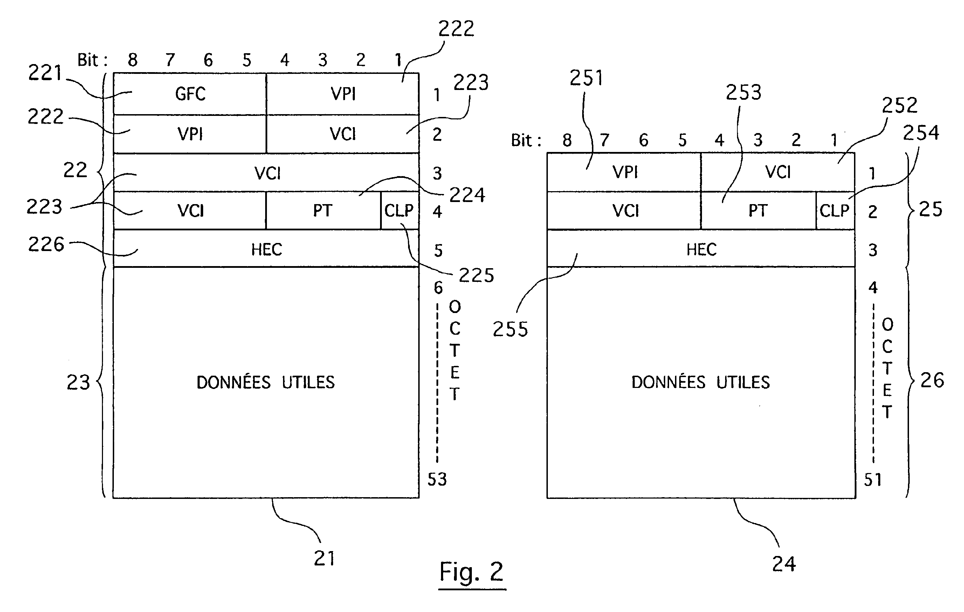

On présente maintenant, en relation avec le schéma de la figure 2, un exemple de cellule réduite selon l'invention en comparaison avec une cellule complète.

De façon classique, une cellule 21 complète comprend un champ 22 en-tête comprenant 5 octets de données relatives à l'adresse du destinataire de la cellule 21, et un champ 23 d'information comprenant 48 octets de données utiles. Le champ 22 en-tête comprend :

- un champ 221 de contrôle de flux générique (ou "GFC" pour "Generic Flow Control" en anglais) codé sur 4 bits. Ce champ 221 a une signification uniquement locale, permettant d'assurer des fonctions locales chez l'usager ;
- deux champs d'adresse, permettant le routage :

  - un champ 222 identifiant le chemin virtuel complet (ou "VPI" pour "Virtual Path Identifier" en anglais) codé sur 8 bits, dont le nombre de bits utilisés peut être convenu préalablement entre un réseau d'un système de transmission mis en oeuvre et un usager du réseau ;
  - un champ 223 identifiant le canal virtuel complet (ou "VCI" pour "Virtual Channel Identifier" en anglais) codé sur 16 bits, dont le nombre de bits utilisés peut également être convenu préalablement entre un réseau d'un système de transmission mis en oeuvre et un usager du réseau ;

- un champ 224 de type d'information (ou "PT" pour "Payload Type" en anglais) codé sur 3 bits. Ce champ 224 indique si la cellule 21 complète comporte une information concernant l'usager ou une information concernant la gestion de la couche associée à la connexion (ou "Connection Associated Layer Management" en anglais) ;
- un champ 225 de priorité à la perte de cellule (ou "CLP" pour "Cell Loss Priority" en anglais) codé sur 1 bit. Ce champ 225 permet d'indiquer, au réseau ATM, s'il peut éliminer la cellule 21 correspondante, lors notamment de la congestion de cellules sur le réseau ATM ;
- un champ 226 de détection et de correction d'erreur (ou "HEC" pour "Header Error Control" en anglais), codé sur 8 bits. Ce champ est utilisé par la couche physique mise en oeuvre pour la détection et la correction d'erreurs dans le champ en-tête de la cellule 21. Ce champ 226 permet d'effectuer une correction sur une unique erreur bit et une détection de plusieurs erreurs bits.

Une cellule 24 réduite, obtenue par la mise en oeuvre du procédé décrit précédemment, comprend, par exemple, un champ 25 en-tête réduit comprenant seulement 3 octets, et un champ 26 d'informations non modifié (comprenant donc 48 octets de données utiles).

Le champ 25 en-tête réduit ne comprend pas le champ de contrôle de flux générique (ou GFC) par rapport au champ 22 en-tête complet. Les équipements de communication reconstruisent le champ de contrôle de flux générique en insérant une série de 4 zéros consécutifs aux emplacements appropriés.

Le champ 25 en-tête réduit comprend :

- deux champs identifiant du multiplexage :

- un champ 251 identifiant de chemin virtuel réduit codé sur 4 bits. Ces 4 bits correspondent aux 4 bits de poids faible du champ 222 identifiant de chemin virtuel complet. En d'autres tenues, on supprime les 4 bits les plus significatifs du champ 222 identifiant de chemin virtuel complet ;
- un champ 252 identifiant de canal virtuel réduit codé sur 8 bits. Ces 8 bits correspondent aux 8 bits de poids faible du champ 223 identifiant de canal virtuel complet. En d'autres termes, on supprime les 8 bits les plus significatifs du champ 223 identifiant de canal virtuel complet.

A réception de ces champs 251 identifiant de chemin virtuel réduit et 252 identifiant de canal virtuel réduit, les équipements de commmunication, qui seront détaillés plus loin, reconstruisent les champs 222 identifiant de chemin virtuel complet et 223 identifiant de canal virtuel complet en insérant 4 et 8 zéros respectivement, en tant que bits de poids fort.

- un champ 253 du type d'information codé sur 3 bits (identique au champ 224 précité). Optionnellement, le champ 253 de type d'information peut être codé sur 2 bits. Un élément de donnée supplémentaire est donc supprimé. Dans ce cas, les équipements de communication reconstruisent l'élément de donnée supprimé, en insérant, un zéro en tant que bit de poids fort. On présente, en annexe I, un mode de réalisation particulier de correspondance entre l'ensemble des valeurs possibles pour un codage du champ 253 de type d'information et leur signification ;
- un champ 254 de priorité de perte de cellule codé sur 1 bit (identique au champ 225 précité). Optionnellement, le champ 254 de priorité de perte de cellule peut être supprimé ;
- un champ 255 de détection et de correction d'erreur située dans le champ 25 en-tête codé sur 8 bits (identique au champ 226 précité). L'algorithme de détection et de correction utilisé ne porte que sur les bits formant le champ 25 en-tête réduit. Selon une variante avantageuse de réalisation, le champ 255 de détection et de correction d'erreur peut présenter une taille réduite, de moins de 8 bits, l'algorithme de détection et de correction utilisé ne portant que sur les bits formant le champ 25 en-tête réduit.

L'exemple de cellule 25 réduite, qui vient d'être décrit, présente un nombre entier d'octets. Cependant, une cellule réduite peut également présenter un nombre de bits d'information compris dans le champ 25 en-tête tel qu'il ne correspond pas à un multiple entier d'octets, tout en restant dans le cadre de la présente invention.

On décrit, ci-dessous, en détail, le champ 255 de détection et de correction d'erreur.

La séquence du champ 255 de détection et de correction d'erreur est générée en divisant le champ d'information 251 à 254 de l'en-tête 25 par un polynôme générateur (H(x)). Le polynôme générateur choisi peut être capable de détecter des erreurs multi-bit et de corriger des erreurs sur un unique bit. Le champ 255 de détection et de correction d'erreur est également utilisé pour délimiter la cellule, à savoir déterminer le début d'une nouvelle cellule correspondant à celui d'un nouveau champ en-tête 25.

Par exemple, un polynôme générateur associé à la cellule 24 réduite correspondant à un champ de détection et de correction d'erreur codé sur 8 bits, qui soit suffisant pour corriger une erreur unique, est le suivant : $1+x+x^2+x^8$.

Le polynôme générateur peut correspondre, au moins en partie, à des codes de Hamming générés afin de corriger la transmission d'une erreur sur un unique bit.

Selon une variante de réalisation, on peut envisager une meilleure correction d'erreur dans le champ 25 en-tête, tout en maintenant la taille du champ 255 de détection et de correction d'erreur.

Les codes utilisés pour la correction d'une seule erreur sont conformes à la règle de Hamming suivante :

$$2^r - r = m + 1 \; ;$$

dans laquelle r est le degré du polynôme générateur et également le nombre de bits du champ 255 de détection et de correction d'erreur, et m est le nombre de bits d'information dans le champ 25 en-tête.

On présente, en annexe II, un premier tableau de correspondance entre la taille du champ 255 de détection et de correction d'erreur et celle du champ 25 en-tête réduit, conformément à la règle de Hamming. On présente, également en annexe II, un deuxième tableau présentant une famille de codes de Hamming en relation avec le degré du polynôme.

En annexe III, différents polynômes générateurs en correspondance avec le degré du polynôme générateur sont présentés dans un tableau.

On donne, ci-après, un exemple de champ 255 de détection et de correction d'erreur réduit pour une cellule 24 comprenant un champ en-tête 25 réduit, au format suivant :

- un champ 251 identifiant de chemin virtuel réduit codé sur 2 bits ;

- un champ 252 identifiant de canal virtuel réduit codé sur 4 bits ;
- un champ 253 de type d'information codé sur 3 bits ;
- un champ 254 de priorité de perte de cellule codé sur 1 bit.

Le nombre de bits d'information du champ 25 en-tête réduit s'élève donc à 10, ce qui correspond à un champ 255 de détection et de correction d'erreur de 5 bits. Le polynôme générateur du champ 255 de détection et de correction d'erreur calculé à l'aide du code de Hamming est, selon le tableau de l'annexe III, le suivant : $1+x^2+x^4+x^5$.

Ce code présente une distance de Hamming de 4, ce qui signifie qu'il n'est capable de corriger que des erreurs d'un unique bit. L'en-tête 25 réduit comprend, dans ce cas, 15 bits. On notera que cet exemple d'en-tête réduit ne présente pas un nombre de bits qui soit un multiple entier d'octets. Pour y remédier, on peut éventuellement utiliser le polynôme générateur de degré 6, pour obtenir, par exemple, un champ 25 en-tête réduit codé sur 16 bits.

Le champ 25 en-tête comprend donc un nombre réduit d'éléments de données du fait que certains des éléments de données du champ 22 en-tête complet ont été supprimés.

On obtient un gain de place, correspondant à un gain en bande passante (ou Gb) que l'on peut exprimer selon la formule suivante :

$$Gb = 100 - (\text{longueur de la cellule 24 réduite} * 100)/(\text{longueur de la cellule 21 complète}).$$

Avec l'exemple décrit en relation avec la figure 2, le gain en bande passante s'élève à 3,77%.

Avec un exemple de cellule réduite au format suivant :

- un champ identifiant de chemin virtuel codé sur 2 bits ;
- un champ identifiant de canal virtuel codé sur 4 bits ;
- un champ du type d'information codé sur 3 bits ;
- un champ de priorité à la perte de cellule codé sur 1 bit ;
- un champ de détection et de correction d'erreur codé sur 5 bits ;
- un champ d'information codé sur 48 octets ;

on obtient un gain en bande passante de 5, 89%.

La cellule 24 réduite est transmise entre deux équipements de communication d'une chaîne de transmission, à savoir un équipement de communication émetteur et un équipement de communication récepteur. Comme on le verra ci-dessous, ces équipements de communication comprennent des moyens de reconstruction des éléments de données supprimés, notamment en insérant des zéros aux emplacements affectés par la suppression d'éléments de données.

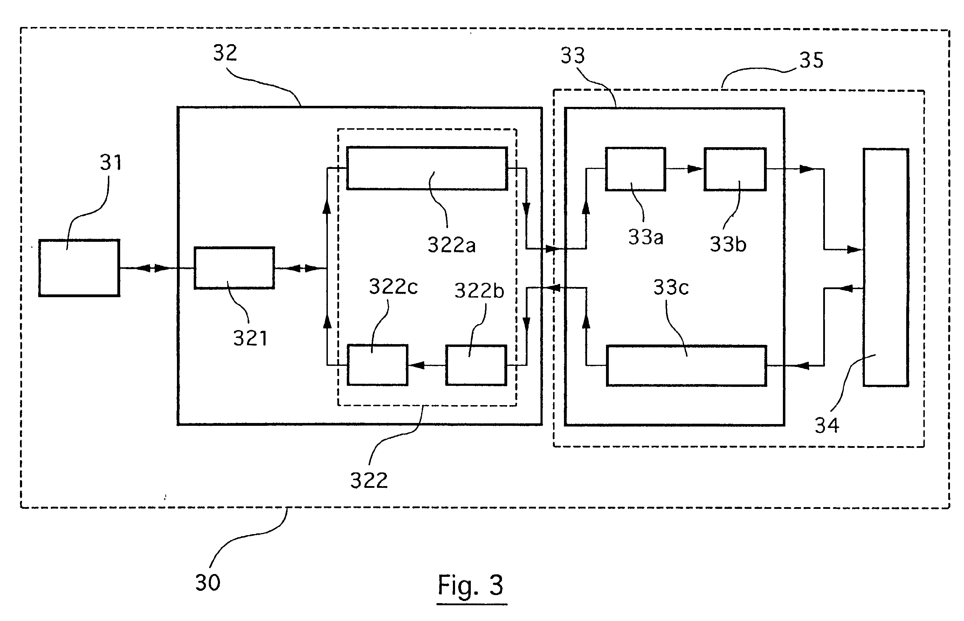

On présente maintenant, en relation avec le schéma simplifié de la figure 3, un mode de réalisation particulier d'un réseau d'un système 30 de transmission de cellules réduites selon l'invention.

De façon classique, le réseau 31 ATM est relié à un réseau 32 de transmission d'extrémité, avec lequel il échange des données organisées sous forme de cellules complètes selon un débit fixe, par exemple de quelques Mbits/s.

On considère, dans la suite de la présente description, que le réseau 32 de transmission d'extrémité est de type filaire mettant en oeuvre la technique SDSL.

On notera cependant que le procédé de l'invention est compatible avec tout type de réseau de transmission. Il peut s'agir notamment des réseaux de transmission de type DSL, ADSL, HDSL...

Ce réseau d'extrémité 32 de transmission comprend une pluralité de moyens 321 de commutation gérant une pluralité de chaînes de transmission terminées par des interfaces d'usager.

Par souci de simplification, seule une chaîne de transmission, comprenant un réseau 32 de transmission, une unité 33 terminale de réseau et une interface 34 d'usager, a été représentée sur la figure 3.

L'étape de réduction (cf figure 1) de la taille des en-têtes est mise en oeuvre sur une ligne 35 d'extrémité qui alimente une unité 34 terminale de réseau.

Les moyens 321 de commutation gèrent d'une part les signaux émis dans le sens descendant, à savoir du réseau 32 de transmission vers l'interface 34 d'usager, et d'autre part les signaux reçus dans le sens montant, à savoir de l'interface 34 d'usager vers le réseau 32 de transmission.

L'interface 34 d'usager, est par exemple un équipement compatible avec la technique de transfert asynchrone, une interface non compatible avec la technique de transfert asynchrone, telle qu'une interface à relai de trame, une interface circuit (T1, E1, V35...), ou une interface Ethernet (marque déposée) utilisant des couches d'adaptation à la technique de transfert asynchrone en haut d'un standard de technique de transfert asynchrone.

D'une façon générale, le sens des signaux échangés est symbolisé par une flèche pointant dans la direction prise par les signaux sur la chaîne de transmission.

Dans une première partie, on décrit les moyens mis en oeuvre dans le sens descendant au sein de la chaîne de transmission.

Le réseau 31 ATM délivre des cellules complètes aux moyens 321 de commutation. Les moyens 321 de commutation commutent les cellules complètes qu'ils reçoivent, en fonction de l'adresse contenue dans le champ en-tête des cellules complètes sur la chaîne de transmission associée.

Selon une variante de réalisation, les moyens 321 de commutation peuvent être remplacés par des moyens de multiplexage, qui sélectionnent une chaîne de transmission parmi plusieurs chaînes de transmission, en fonction de l'adresse contenue dans le champ en-tête des cellules complètes qu'ils reçoivent.

Les moyens 321 de commutation délivrent ces cellules complètes à des moyens 322 de traitement de cellules qui comprennent des moyens 322a de réduction de la taille d'au moins certains en-têtes complets des cellules complètes. Ces moyens 322a de réduction suppriment les éléments de données non utilisés d'au moins un champ compris dans le champ en-tête complet de certaines cellules complètes reçues, de façon à former des en-têtes réduits afin de construire des cellules réduites.

De façon avantageuse, les moyens 322 de traitement de cellules décident, lors de l'établissement d'une liaison et/ou en cours de transmission, de la mise en oeuvre de l'étape de réduction et/ou des conditions de mise en oeuvre correspondantes. Cela permet de gérer plus efficacement la situation, en fonction des besoins et des ressources disponibles.

Les conditions de mise en oeuvre de l'étape de réduction comprennent, par exemple, la taille des différents champs compris dans le champ en-tête des cellules réduites.

Selon une variante de réalisation, la mise en oeuvre de l'étape de réduction et/ou des conditions de mise en oeuvre de l'étape de réduction est négociée entre les moyens 322 de traitement des cellules et l'unité 33 terminale de réseau en cours de transmission. Une telle mise en oeuvre peut être opérée, lorsque la négociation est simple par exemple. Lorsque cette négociation est complexe, on peut envisager de faire appel à des couches supérieures apportant de "l'intelligence" et/ou la "réflexion" nécessaire(s). Cela permet notamment de s'adapter aux performances de l'unité 33 terminale du réseau correspondante et de tenir compte du taux d'encombrement en cellules échangées en attente devant les équipements de communication.

Selon une variante de réalisation, l'étape de réduction de la taille des en-têtes est mise en oeuvre, de façon systématique, entre les moyens 322 de traitement des cellules du réseau 32 de transmission et l'unité 33 terminale de réseau.

Les moyens 322a de réduction alimentent, en cellules réduites et/ou en cellules complètes, une unité 33 terminale de réseau.

L'unité 33 terminale de réseau comprend :

- des moyens 33a de synchronisation, qui recherchent le début des cellules complètes et/ou des cellules réduites, permettant notamment de déterminer les décisions prises par les moyens 322 de traitement des cellules.
- des moyens 33b de reconstruction des en-têtes complets, qui, d'une part, reconstruisent des cellules complètes, en insérant des zéros, aux endroits des éléments de données supprimés, au sein des champs en-têtes réduits des cellules affectées par la réduction, et d'autre part recalculent le champ 226 de détection et de correction d'erreur associé au champ en-tête complet reconstruit.

Optionnellement, on peut prévoir des moyens supplémentaires de synchronisation bit. Ces moyens de synchronisation bit permettent de rechercher le passage d'un élément de donnée à son suivant. Cela permet notamment de localiser les endroits où l'on a préalablement opéré la suppression d'éléments de données, pour effectuer un remplissage par des éléments de données, afin de reconstruire les cellules complètes.

Les moyens 33b de reconstruction des en-têtes délivrent des cellules complètes à l'interface 34 d'usager. Cette interface 34 d'usager peut recevoir, en conséquence, plus de données utiles que si elle recevait uniquement des cellules complètes. Cela lui permet de mettre en oeuvre, par exemple simultanément, des services supplémentaires tels qu'un ou des service(s) de ligne louée, un ou des service(s) de relais de trames et/ou un ou des service(s) liés à Internet (marque déposée).

On notera que les moyens mis en oeuvre en aval de l'équipement 33 de communication récepteur des cellules réduites ne nécessitent aucune modification pour pouvoir traiter des cellules réduites (puisque l'équipement 33 de communication récepteur des cellules réduites est équipé de moyens de reconstruction des en-têtes complets assurant la reconversion des cellules réduites en cellules complètes).

On décrit maintenant les moyens mis en oeuvre dans le sens montant au sein de la chaîne de transmission.

On suppose ici que l'interface 34 d'usager peut émettre en direction du réseau 32 de transmission des données organisées sous forme de cellules complètes. Pour ce faire, sur une ligne d'extrémité, l'interface 34 d'usager alimente en cellules complètes, l'unité 33 terminale de réseau. Bien sûr, selon un autre mode de réalisation, on peut construire directement des cellules réduites.

Selon une caractéristique essentielle de l'invention, l'unité 33 terminale de réseau comprend des moyens 33c de réduction de la taille d'au moins certains en-têtes complets. Ces moyens 33c de réduction suppriment les éléments de données non utilisés, par exemple dans les mêmes conditions, que celles mises en oeuvre dans le sens descendant, à savoir au sein du champ en-tête des cellules complètes, de façon à former des en-têtes réduits et élaborer ainsi des cellules réduites. Les moyens 33c de réduction délivrent des cellules réduites aux moyens 322 de traitement des cellules du réseau 32 de transmission.

Avantageusement, les moyens 322 de traitement des cellules comprennent :

-   des moyens 322b de synchronisation, qui repèrent, lors de l'établissement et/ou en cours de transmission, le début de chaque cellule reçue, permettant de déterminer les décisions prises par l'unité 33 terminale de réseau pour la mise en oeuvre de l'étape de réduction de la taille des en-têtes.
-   des moyens 322c de reconstruction des en-têtes complets. Ces moyens 322c de reconstruction des en-têtes complets reconstruisent, à partir des en-têtes réduits des cellules réduites, des cellules complètes, en insérant aux endroits appropriés des zéros, et recalculent le champ de détection et de correction d'erreur associé au champ en-tête complet reconstruit.

Les moyens 322c de reconstruction délivrent des cellules complètes aux moyens 321 de commutation. Ces derniers alimentent, en fonction de l'adresse du ou des destinataire(s) comprise dans le champ en-tête des cellules complètes, la chaîne de transmission adéquate.

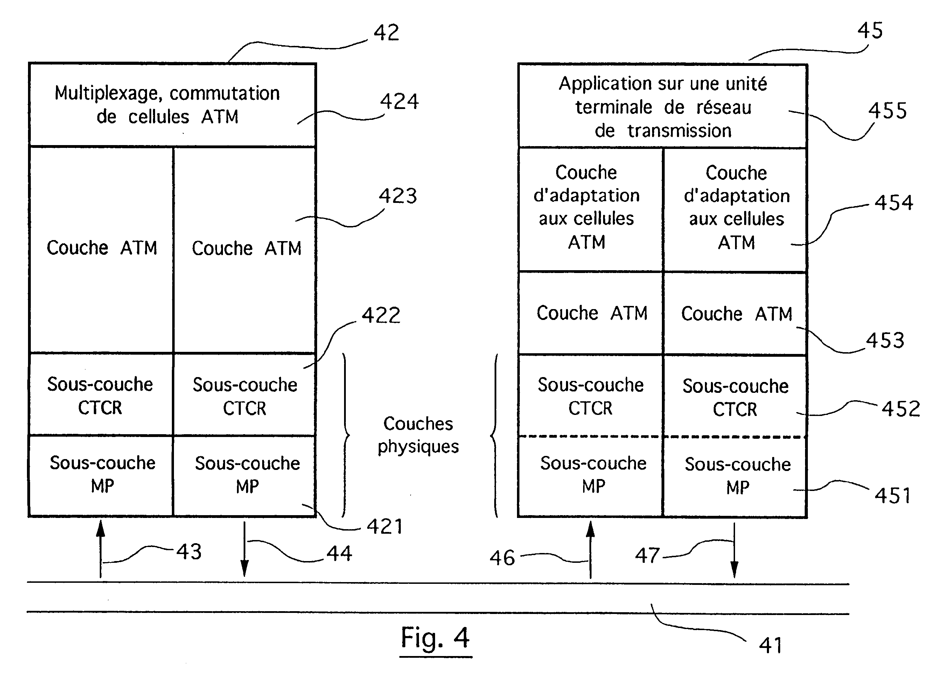

On présente maintenant, en relation avec le schéma simplifié de la figure 4, un mode de réalisation particulier d'un ensemble fonctionnel d'adaptation au procédé selon l'invention de transmission de données organisées en cellules.

On suppose tout d'abord que l'on met en oeuvre un réseau 41 de transmission fonctionnant selon un débit relativement faible, d'environ quelques Mbits/s.

De façon classique, on effectue, au sein du réseau 32 de transmission des données organisées sous forme de cellules complètes, parmi un ensemble 42 de fonctions, une pluralité de fonctions qui comprennent, de manière identique, pour les sens montant 43 et descendant 44,

-   une première couche 423 ATM de présentation (définissant les informations échangées) selon la technique de transfert asynchrone ;
-   une seconde couche 424 d'application (définissant les mécanismes communs aux applications et la signification des données échangées) : multiplexage, commutation de cellules ATM.

Ces fonctions 423, 424 sont connues et ne sont donc pas décrites plus en détail.

Egalement de façon classique, l'ensemble fonctionnel 42 comprend une première sous-couche 421 de milieu physique MP, qui réalise des fonctions dépendant du milieu physique (c'est-à-dire du réseau 41 de transmission et de la ligne 35 d'extrémité).

Cette fonction 421 est également connue de l'homme du métier et ne sera donc pas détaillée.

L'ensemble fonctionnel 42 comprend une deuxième sous-couche physique de convergence de transmission de cellules réduites CTCR 422, qui vient s'intégrer entre la première sous-couche MP 421 et la couche ATM 423. Conformément à l'invention, cette seconde sous-couche physique 422 permet de convertir le flux de cellules complètes arrivant de la première couche ATM 423 en un flux de cellules réduites que l'on peut transmettre à la première sous-couche 421 MP.

Par ailleurs, on notera que cette sous-couche physique de convergence de transmission de cellules réduites CTCR 422 réutilise également les mêmes techniques qu'une sous-couche physique de convergence de transmission classique (pour TC "Transmission Convergence" en anglais) définie par la norme ATM (située entre la couche ATM et la sous-couche du Milieu Physique MP).

De façon classique, on effectue, au sein de la ligne 35 d'extrémité, parmi un ensemble 45 de fonctions, une pluralité de fonctions qui comprennent, notamment, de manière symétrique, pour les sens montant 46 et descendant 47 :

-   une première couche 453 ATM de présentation (définissant les informations échangées) selon la technique de transfert asynchrone ;
-   une seconde couche 454 d'adaptation aux cellules ATM ;
-   une troisième couche 455 d'application (définissant les mécanismes communs aux applications et la signification des données échangées) : applications sur une interface 34 d'usager du réseau de transmission.

Ces fonctions 453, 454, 455 sont connues et ne sont donc pas décrites plus en détail.

Egalement de façon classique, l'ensemble fonctionnel 45 comprend une première sous-couche 451 de milieu physique MP, qui réalise des fonctions dépendant du milieu physique.

Cette fonction 451 est également connue de l'homme du métier et ne sera donc pas détaillée.

L'ensemble fonctionnel 45 comprend une deuxième sous-couche physique de convergence de transmission de cellules réduites CTCR 452, qui vient s'intégrer entre la première sous-couche MP 451 et la couche ATM 453. Selon l'invention, cette seconde sous-couche physique permet de convertir le flux de cellules complètes arrivant de la première couche ATM 453 en un flux de cellules réduites que l'on peut transmettre à la première sous-couche 451 MP.

On notera que cette sous-couche physique de convergence de transmission de cellules réduites CTCR 422 réutilise également les mêmes techniques qu'une sous-couche physique de convergence de transmission classique définie par la norme ATM.

Il convient de noter qu'aucune modification de la troisième couche 455 d'application et/ou de la deuxième couche 454 d'adaptation aux cellules ATM et/ou de la première couche 453 ATM n'est à effectuer. Cela permet d'adapter de manière aisée une unité terminale de réseau au procédé de l'invention.

### ANNEXE I

### Mode de réalisation particulier de la correspondance entre le codage du champ 253 du type d'information de cellules réduites et la signification associée

On présente, ci-après, un tableau illustrant un mode de réalisation particulier de la correspondance entre les différents codes possibles pour le champ 253 du type d'information du champ en-tête 25 réduit et leur signification.

| Codage du champ 253 de type d'information (le bit le plus significatif étant placé devant) | Signification |
|---|---|
| 00 | Cellule de données relative à l'usager, type d'Unité de Donnée de Service = 0 |
| 01 | Cellule de données relative à l'usager, type d'Unité de Donnée de Service = 1 |
| 10 | Cellule relative au flux F5 Opération, Administration, Maintenance (OAM) par Segment |
| 11 | Cellule relative au flux F5 Opération, Administration, Maintenance (OAM) d'extrémité à extrémité |

### ANNEXE II

### Mode de réalisation particulier de correspondance entre la taille du champ de détection et de correction d'erreur, la taille maximale du champ information de l'en-tête et la taille de l'en-tête résultant

On présente, ci-dessous, un tableau présentant différentes tailles du champ 255 de détection et de correction d'erreur en correspondance avec la taille maximale du champ information de l'en-tête et de l'en-tête résultant.

| | | | | | | |
|---|---|---|---|---|---|---|
| Taille maximale du champ information de l'en-tête (en nombre de bits) correspondant à m dans la règle de Hamming | 1 | 4 | 11 | 26 | 57 | 120 |
| Champ 255 de détection et de correction d'erreur permettant de protéger les informations dont la taille est inférieure ou égale à m, correspondant à r dans la règle de Hamming | 2 | 3 | 4 | 5 | 6 | 7 |
| Longueur maximale de l'en-tête (en nombre de bits) correspondant à m+r | 3 | 7 | 15 | 31 | 63 | 127 |

On notera que, lorsque le nombre de bits d'information du champ en-tête ne correspond pas aux valeurs ci-dessus, il faut choisir le nombre de bits (désigné par r dans la règle de Hamming) du champ 255 de détection et de correction d'erreur entier immédiatement supérieur à la valeur (r), respectant la règle de Hamming.

Dans le tableau suivant, on présente une famille de codes de Hamming correspondant au degré du polynôme générateur compris entre 3 et 7.

| Degré du polynôme générateur (r) | Polynôme générateur H(x) |
|---|---|
| 3 | $1+x+x^3$ |
| 4 | $1+x+x^4$ |
| 5 | $1+x^2+x^5$ |
| 6 | $1+x+x^6$ |
| 7 | $1+x^3+x^7$ |
| 7 | $1+x^2+x^3+x^4+x^5+x^6+x^7$ |

Si deux erreurs se produisent, alors l'algorithme de correction introduira une troisième erreur. Une solution, pour s'en protéger, consiste à être capable de détecter des nombres impairs d'erreurs. Pour ce faire, on peut, par exemple, multiplier le polynôme générateur développé ci-dessus par $(1+x)$. Cela augmente le nombre de vérification(s) de bit(s) d'une unité, de sorte que le nombre maximum d'information(s) du champ en-tête réduit doit, simultanément, être réduit d'une unité, pour rester conforme à la théorie de code d'Abramson. La règle de Hamming devient alors la suivante : $2^r - r = m + 2$ cette formule étant appelée par la suite, règle de Hamming modifiée.

Dans la mesure du possible, les polynômes générateurs du champ 255 de détection et de correction d'erreur doivent correspondre au nombre de bits d'information du champ en-tête réduit.

**ANNEXE III**

**Mode de réalisation particulier d'une correspondance entre la taille du champ 255 de détection et de correction d'erreur, des polynômes générateurs, la taille maximale du champ d'information de l'en-tête réduit et du champ en-tête réduit résultant**

On présente, ci-après, un tableau illustrant un mode de réalisation d'une correspondance entre certains polynômes générateur, la taille du champ 255 de détection et de correction d'erreur, la taille maximale du champ d'information de l'en-tête associé et du champ en-tête résultant.

| | 35 | 30 | 25 | 20 | 15 | 10 | 5 |
|---|---|---|---|---|---|---|---|
| Champ du 255 de détection et de correction d'erreur (bits) permettant de protéger les informations dont la taille est inférieure ou égale à m, correspondant à r dans la règle de Hamming modifiée (cf p.22) | 4 | 5 | 6 | 7 | 8 | | 8 |
| Exemple de Polynome générateur (1+x)*H(x) | $1+x^2+x^3+x^4$ | $1+x^2+x^4+x^5$ | $1+x+x^2+x^3+x^5+x^6$ | $1+x^2+x^6+x^7$ | $1+x+x^3+x^4+x^7+x^8$ | | $1+x+x^2+x^8$ (ATM) |
| Taille de l'en-tête (en nombre de bits) égale à m+r | 7 | 15 | 31 | 63 | 127 | | 127 |
| Taille maximale du champ information de l'en-tête (en nombre de bits) correspondant à m dans la règle de Hamming modifiée (cf p.22) | 3 | 10 | 25 | 56 | 119 | | 119 |

**EP 0 987 917 B1**

**Revendications**

1. Procédé de transmission de données organisées en cellules (21) comprenant chacune un en-tête (22), dit en-tête complet, et un champ (23) de type d'information, ledit en-tête (22) complet comprenant un identifiant (222) de chemin virtuel complet codé sur 8 bits et un identifiant (223) de canal virtuel complet codé sur 16 bits, **caractérisé en ce qu'**il comprend une étape (14) de réduction de la taille dudit en-tête (22) complet, entre deux équipements (32, 33) de communication d'une chaîne de transmission, pour former une cellule (24) réduite avec un en-tête (25) réduit comprenant un identifiant (251) de chemin virtuel réduit codé sur 4 bits, correspondant aux 4 bits de poids faible dudit identifiant (222) de chemin virtuel complet, et un identifiant (252) de canal virtuel réduit codé sur 8 bits, correspondant aux 8 bits de poids faible dudit identifiant (223) de canal virtuel complet, lesdits équipements (32, 33) reconstruisant lesdits identifiants (222, 223) complets par insertion de 4 et 8 zéros respectivement pour former les bits de poids fort.

2. Procédé selon la revendication 1, **caractérisé en ce que** ladite étape (14) de réduction de la taille des en-têtes (22) est mise en oeuvre sur une ligne (35) d'extrémité, alimentant une unité (33) terminale du réseau.

3. Procédé selon l'une quelconque des revendication 1 et 2, **caractérisé en ce que** ledit en-tête (22) complet comprenant un champ (221) de contrôle de flux générique de 4 bits, ledit champ (221) de contrôle de flux générique est supprimé dans ledit en-tête (25) réduit, lesdits équipement (32, 33) reconstruisant ledit champ (221) de contrôle de flux générique par insertion de 4 zéros.

4. Procédé selon l'une quelconque des revendications 1 à 3, **caractérisé en ce que**, ledit en-tête (25) réduit et ledit en-tête (22) complet comprennent un champ (226) de détection et de correction d'erreur codé sur 8 bits, l'algorithme de détection et de correction ne portant que sur les bits formant ledit en-tête (25) réduit.

5. Procédé selon l'une quelconque des revendications 1 à 4, **caractérisé en ce que**, ledit en-tête (22) complet comprenant un champ (226) de détection et de correction d'erreur codé sur 8 bits, ledit en-tête réduit comprend un champ de détection et de correction d'erreur réduit, de moins de 8 bits, l'algorithme de détection et de correction ne portant que sur les bits formant ledit en-tête réduit.

6. Procédé selon la revendication 5, **caractérisé en ce que** ledit champ de détection et de correction réduit comprend 5 bits et est calculé à l'aide d'un code de Hamming.

7. Procédé selon l'une quelconque des revendications 1 à 6, **caractérisé en ce que** ladite étape (14) de réduction est mise en oeuvre, de façon systématique, entre deux équipements (32, 33) prédéterminés.

8. Procédé selon l'une quelconque des revendications 1 à 6, **caractérisé en ce qu'**un premier desdits équipements (32, 33) décide, lors de l'établissement d'une liaison et/ou en cours de transmission, de la mise en oeuvre de ladite étape (14) de réduction et/ou des conditions de mise en oeuvre correspondantes, le second desdits équipements (32, 33) adoptant ces décisions.

9. Procédé selon l'une quelconque des revendications 1 à 7, **caractérisé en ce que** la mise en oeuvre de ladite étape (14) de réduction et/ou des conditions de mise en oeuvre de ladite étape (14) de réduction est négociée entre lesdits équipements (32, 33) en cours de transmission.

10. Procédé selon la revendication 9, **caractérisé en ce que** lesdites conditions de mise en oeuvre comprennent la taille d'au moins un champ (251, 252, 253, 254, ou 255) dudit en-tête (25) réduit.

11. Procédé selon l'une quelconque des revendications 1 à 10, **caractérisé en ce qu'**il comprend une étape de synchronisation, dans chacun desdits équipements (32, 33), consistant à rechercher le début de cellules (21) complètes et/ou de cellules (24) réduites.

12. Procédé selon la revendication 11, **caractérisé en ce que** ladite étape de synchronisation permet la détermination de décisions dudit premier équipement (32,33).

13. Procédé selon l'une quelconque des revendications 1 à 12, **caractérisé en ce qu'**il est mis en oeuvre au sein d'un système (30) de communication au standard ATM.

**14.** Cellule réduite d'un signal de données organisées en cellules comprenant chacune un en-tête réduit et un champ de type d'information, correspondant à une cellule d'origine comprenant un en-tête complet et un champ de type d'information, ledit en-tête (22) complet comprenant un identifiant (222) de chemin virtuel complet codé sur 8 bits et un identifiant (223) de canal virtuel complet codé sur 16 bits,

**caractérisé en ce que** ledit en-tête (25) réduit comprend un identifiant (251) de chemin virtuel réduit codé sur 4 bits, correspondant aux 4 bits de poids faible dudit identifiant (222) de chemin virtuel complet, et un identifiant (252) de canal virtuel réduit codé sur 8 bits, correspondant aux 8 bits de poids faible dudit identifiant (223) de canal virtuel complet,

et **en ce qu'**elle est destinée à être transmise entre deux équipements comprenant de moyens de reconstruction desdits identifiants (222, 223) complets par insertion de 4 et 8 zéros respectivement pour former les bits de poids fort.

**15.** Système (30) de communication mettant en oeuvre des cellules comprenant chacune un en-tête (22), dit en-tête complet, et un champ (23) de type d'information, ledit en-tête (22) complet comprenant un identifiant (222) de chemin virtuel complet codé sur 8 bits et un identifiant (223) de canal virtuel complet codé sur 16 bits, ledit système comprenant une pluralité d'équipement (32, 33) de communication,

**caractérisé en ce qu'**au moins deux desdits équipements (32, 33) comprennent des moyens adaptés à échanger au moins une cellule comprenant un en-tête (25) réduit, comprenant un identifiant (251) de chemin virtuel réduit codé sur 4 bits, correspondant aux 4 bits de poids faible dudit identifiant (222) de chemin virtuel complet, et un identifiant (252) de canal virtuel réduit codé sur 8 bits, correspondant (223) aux 8 bits de poids faible dudit identifiant de canal virtuel complet,

lesdits équipements (32, 33) comprenant aussi des moyens (322e, 33b) de reconstruction desdits identifiants (222, 223) complets, à partir dudit en-tête (25) réduit, par insertion de 4 et 8 zéros respectivement pour former les bits de poids fort.

**16.** Unité (33) terminale de réseau d'un système (30) de communication selon la revendication 15, **caractérisé en ce qu'**elle comprend des moyens (33c) de réduction de la taille d'un en-tête (22) complet, pour former un en-tête (25) réduit comprenant un identifiant (251) de chemin virtuel réduit codé sur 4 bits, correspondant aux 4 bits de poids faible d'un identifiant (222) de chemin virtuel complet, et un identifiant (252) de canal virtuel réduit codé sur 8 bits, correspondant aux 8 bits de poids faible d'un identifiant (223) de canal virtuel complet,

et des moyens (33b) de reconstruction d'un en-tête (22) complet, à partir d'un en-tête (25) réduit, par insertion de 4 et 8 zéros respectivement pour former les bits de poids fort.

**Claims**

**1.** Method for transmitting data organised in cells (21) each comprising a header (22) called a complete header, and an information type field (23), said complete header (22) including a complete virtual path identifier (222) coded on 8 bits and a complete virtual channel identifier (223) coded on 16 bits,

**characterised in that** it includes a step (14) to reduce the size of said complete header (22) between two items of communication equipment (32, 33) in a transmission chain to form a small cell (24) with a reduced header (25) including a reduced virtual path identifier (251) coded on 4 bits corresponding to the four low order bits of said complete path identifier (222) and a reduced virtual channel identifier (252) coded on 8 bits, corresponding to the 8 low order bits of said complete virtual channel identifier (223), said items of equipment (32, 33) reconstructing said complete identifiers (222, 223) by insertion of 4 and 8 zeros respectively to form the high order bits.

**2.** Method set forth in claim 1, **characterised in that** said step (14) to reduce the size of the headers (22) is used on one end line (35), supplying power to a terminal unit (33) of the network.

**3.** Method set forth in either of claims 1 and 2, **characterised in that** said complete header (22) comprising a generic 4-bit flow control field (221), said generic flow control field (221) is deleted in said reduced header (25), said items of equipment (32, 33) rebuilding said generic flow control field (221) by the insertion of 4 zeros.

**4.** Method set forth in any one of claims 1 to 3, **characterised in that** said reduced header (25) and said complete header (22) include an error detection and correction field (226) coded on 8 bits, the detection and correction algorithm only applying to the bits forming said reduced header (25).

**5.** Method set forth in any one of claims 1 to 4, **characterised in that** said complete header (22) comprising an error detection and correction field (226) coded on 8 bits, said reduced header comprises a reduced error detection and correction field with at least 8 bits, the detection and correction algorithm only applying to bits forming said reduced header.

6. Method set forth in claim 5, **characterised in that** said reduced detection and correction field comprises 5 bits and is calculated using a Hamming code.

7. Method set forth in any one of claims 1 to 6, **characterised in that** said reduction step (14) is systematically used between two predetermined items of equipment (32, 33).

8. Method set forth in any one of claims 1 to 6, **characterised in that** a first of said items of equipment (32, 33) decides on use of said reduction step (14) and / or corresponding implementation conditions when a connection is being set up and / or during transmission, the second of said items of equipment (32, 33) adopting these decisions.

9. Method set forth in any one of claims 1 to 7, **characterised in that** use of said reduction step (14) and / or implementation conditions of said reduction step (14) is negotiated between said items of equipment (32, 33) during transmission.

10. Method set forth in claim 9, **characterised in that** said implementation conditions include the size of at least one field (251, 252, 253, 254 or 255) of said reduced header (25).

11. Method set forth in any one of claims 1 to 10, **characterised in that** it includes a synchronisation step in each of said equipment (32, 33) consisting of searching for the beginning of complete cells (21) and / or reduced cells (24).

12. Method set forth in claim 11, **characterised in that** said synchronisation step is used to determine decisions of said first equipment (32, 33).

13. Method set forth in any one of claims 1 to 12, **characterised in that** it is used within a communication system (30) to the ATM standard.

14. Reduced cell of signal of data organised in cells each including a reduced header and an information type field, corresponding to an original cell comprising a complete header and an information type field, said complete header (22) including a complete virtual path identifier (222) coded on 8 bits and a complete virtual channel identifier (223) coded on 16 bits,
**characterised in that** said reduced header (25) includes a reduced virtual path identifier (251) coded on 4 bits corresponding to the 4 low order bits of said complete virtual path identifier (222) and a reduced virtual channel identifier (252) coded on 8 bits, corresponding to the 8 low order bits of said complete virtual channel identifier (223), and **in that** it will be transmitted between two items of equipment including means of reconstruction of said complete identifiers (222, 223) by insertion of 4 and 8 zeros respectively to form the high order bits.

15. Communication system (30) using cells each including a header (22) called the complete header, and an information type field (23), said complete header (22) including a complete virtual path identifier (222) coded on 8 bits and a complete virtual channel identifier (223) coded on 16 bits, said system including a plurality of communication equipment (32, 33),
**characterised in that** at least two of said items of equipment (32, 33) include means adapted to exchange at least one cell including a reduced header (25), including a reduced virtual path identifier (251) coded on 4 bits, corresponding to the 4 low order bits of said complete virtual path identifier (222), and a reduced virtual channel identifier (252) coded on 8 bits, corresponding (223) to the 8 low order bits of said complete virtual channel identifier, said items of equipment (32, 33) also including means (322c, 33b) of reconstructing said complete identifiers (222, 223), using said reduced header (25), by insertion of 4 and 8 zeros respectively to form the high order bits.

16. Network terminal unit (33) of a communication system (30) set forth in claim 15, **characterised in that** it includes means (33c) of reducing the size of a complete header (22) to form a reduced header (25) comprising a reduced virtual path identifier (251) coded on 4 bits, corresponding to the 4 low order bits of a complete virtual path identifier (222) and a reduced virtual channel identifier (252) coded on 8 bits, corresponding to the 8 low order bits of a complete virtual channel identifier (223),
and means (33b) of reconstruction of a complete header (22) from a reduced header (25), by insertion of 4 and 8 zeros respectively to form the high order bits.

**Patentansprüche**

1. Verfahren zur Übertragung von Daten, die in Zellen (21) organisiert sind, welche jeweils einen Header (22), der als vollständiger Header bezeichnet wird, und ein Feld (23) vom Typ Informationsfeld umfassen, wobei der vollständige Header (22) eine in 8 Bits codierte vollständige Kennung (222) des virtuellen Pfades und eine in 16 Bits codierte vollständige Kennung (223) des virtuellen Kanals umfasst, **dadurch gekennzeichnet, dass** es einen Schritt (14) der Verringerung der Größe des vollständigen Headers (22) zwischen zwei Kommunikationseinrichtungen (32, 33) einer Übertragungskette umfasst, um eine reduzierte Zelle (24) mit einem reduzierten Header (25) zu bilden, der eine in 4 Bits codierte reduzierte Kennung (251) des virtuellen Pfades, welche den 4 niederwertigen Bits der vollständigen Kennung (222) des virtuellen Pfades entspricht, und eine in 8 Bits codierte reduzierte Kennung (252) des virtuellen Kanals, welche den 8 niederwertigen Bits der vollständigen Kennung (223) des virtuellen Kanals entspricht, umfasst, wobei die Einrichtungen (32, 33) die vollständigen Kennungen (222, 223) durch Einfügung von 4 bzw. 8 Nullen, um die höherwertigen Bits zu bilden, rekonstruieren.

2. Verfahren nach Anspruch 1, **dadurch gekennzeichnet, dass** der Schritt (14) der Verringerung der Größe der Header (22) auf einer Endleitung (35) durchgeführt wird, die ein Endgerät (33) des Netzes speist.

3. Verfahren nach einem der Ansprüche 1 und 2, **dadurch gekennzeichnet, dass**, wenn der vollständige Header (22) ein Feld (221) der generellen Flusssteuerung von 4 Bits umfasst, das Feld (221) der generellen Flusssteuerung in dem reduzierten Header (25) weggelassen wird, wobei die Einrichtungen (32, 33) das Feld (221) der generellen Flusssteuerung durch Einfügung von 4 Nullen rekonstruieren.

4. Verfahren nach einem der Ansprüche 1 bis 3, **dadurch gekennzeichnet, dass**, wenn der reduzierte Header (25) und der vollständige Header (22) ein in 8 Bits codiertes Feld (226) der Fehlererkennung und -korrektur umfassen, der Erkennungs- und Korrekturalgorithmus sich nur auf die Bits erstreckt, die den reduzierten Header (25) bilden.

5. Verfahren nach einem der Ansprüche 1 bis 4, **dadurch gekennzeichnet, dass**, wenn der vollständige Header (22) ein in 8 Bits codiertes Feld (226) der Fehlererkennung und -korrektur umfasst, der reduzierte Header ein reduziertes Feld der Fehlererkennung und -korrektur von weniger als 8 Bits umfasst, wobei der Erkennungs- und Korrekturalgorithmus sich nur auf die Bits erstreckt, die den reduzierten Header bilden.

6. Verfahren nach Anspruch 5, **dadurch gekennzeichnet, dass** das reduzierte Feld der Fehlererkennung und -korrektur 5 Bits umfasst und mit Hilfe eines Hammingcodes berechnet wird.

7. Verfahren nach einem der Ansprüche 1 bis 6, **dadurch gekennzeichnet, dass** der Schritt (14) der Verringerung systematisch zwischen zwei vorgegebenen Einrichtungen (32, 33) durchgeführt wird.

8. Verfahren nach einem der Ansprüche 1 bis 6, **dadurch gekennzeichnet, dass** eine erste der Einrichtungen (32, 33) bei der Herstellung einer Verbindung und/oder während einer Übertragung über die Durchführung des Schrittes (14) der Verringerung und/oder die entsprechenden Bedingungen der Durchführung entscheidet, wobei die zweite der Einrichtungen (32; 33) diese Entscheidungen annimmt.

9. Verfahren nach einem der Ansprüche 1 bis 7, **dadurch gekennzeichnet, dass** die Durchführung des Schrittes (14) der Verringerung und/oder Bedingungen der Durchführung des Schrittes (14) der Verringerung zwischen den Einrichtungen (32, 33) während der Übertragung ausgehandelt werden.

10. Verfahren nach Anspruch 9, **dadurch gekennzeichnet, dass** die Bedingungen der Durchführung die Größe wenigstens eines Feldes (251, 252, 253, 254 oder 255) des reduzierten Headers (25) umfassen.

11. Verfahren nach einem der Ansprüche 1 bis 10, **dadurch gekennzeichnet, dass** es einen Synchronisationsschritt in jeder der Einrichtungen (32, 33) umfasst, der darin besteht, den Anfang von vollständigen Zellen (21) und/oder von reduzierten Zellen (24) zu suchen.

12. Verfahren nach Anspruch 11, **dadurch gekennzeichnet, dass** der Synchronisationsschritt die Bestimmung von Entscheidungen der ersten Einrichtung (32, 33) ermöglicht.

13. Verfahren nach einem der Ansprüche 1 bis 12, **dadurch gekennzeichnet, dass** es innerhalb eines Kommunikationssystems (30) nach ATM-Standard durchgeführt wird.

**14.** Reduzierte Zelle eines Signals von Daten, die in Zellen organisiert sind, welche jeweils einen reduzierten Header und ein Feld vom Typ Informationsfeld umfassen, die einer Ursprungszelle entspricht, die einen vollständigen Header und ein Feld vom Typ Informationsfeld umfasst, wobei der vollständige Header (22) eine in 8 Bits codierte vollständige Kennung (222) des virtuellen Pfades und eine in 16 Bits codierte vollständige Kennung (223) des virtuellen Kanals umfasst,

**dadurch gekennzeichnet, dass** der reduzierte Header (25) eine in 4 Bits codierte reduzierte Kennung (251) des virtuellen Pfades, welche den 4 niederwertigen Bits der vollständigen Kennung (222) des virtuellen Pfades entspricht, und eine in 8 Bits codierte reduzierte Kennung (252) des virtuellen Kanals, welche den 8 niederwertigen Bits der vollständigen Kennung (223) des virtuellen Kanals entspricht, umfasst,

und **dadurch**, dass sie dazu bestimmt ist, zwischen zwei Einrichtungen übertragen zu werden, welche Mittel zur Rekonstruktion der vollständigen Kennungen (222, 223) durch Einfügung von 4 bzw. 8 Nullen, um die höherwertigen Bits zu bilden, umfassen.

**15.** Kommunikationssystem (30), in dem Zellen verwendet werden, welche jeweils einen Header (22), der als vollständiger Header bezeichnet wird, und ein Feld (23) vom Typ Informationsfeld umfassen, wobei der vollständige Header (22) eine in 8 Bits codierte vollständige Kennung (222) des virtuellen Pfades und eine in 16 Bits codierte vollständige Kennung (223) des virtuellen Kanals umfasst, wobei das System mehrere Kommunikationseinrichtungen (32, 33) umfasst,

**dadurch gekennzeichnet, dass** wenigstens zwei der Einrichtungen (32, 33) Mittel umfassen, die in der Lage sind, wenigstens eine Zelle auszutauschen, die einen reduzierten Header (25) umfasst, der eine in 4 Bits codierte reduzierte Kennung (251) des virtuellen Pfades, welche den 4 niederwertigen Bits der vollständigen Kennung (222) des virtuellen Pfades entspricht, und eine in 8 Bits codierte reduzierte Kennung (252) des virtuellen Kanals, welche den 8 niederwertigen Bits der vollständigen Kennung (223) des virtuellen Kanals entspricht, umfasst,

wobei die Einrichtungen (32, 33) außerdem Mittel (322c, 33b) zur Rekonstruktion der vollständigen Kennungen (222, 223) ausgehend von dem reduzierten Header (25) durch Einfügung von 4 bzw. 8 Nullen, um die höherwertigen Bits zu bilden, umfassen.

**16.** Netzendgerät (33) eines Kommunikationssystems (30) nach Anspruch 15,

**dadurch gekennzeichnet, dass** es umfasst: Mittel (33c) zur Verringerung der Größe des vollständigen Headers (22), um einen reduzierten Header (25) zu bilden, der eine in 4 Bits codierte reduzierte Kennung (251) des virtuellen Pfades, welche den 4 niederwertigen Bits einer vollständigen Kennung (222) des virtuellen Pfades entspricht, und eine in 8 Bits codierte reduzierte Kennung (252) des virtuellen Kanals, welche den 8 niederwertigen Bits einer vollständigen Kennung (223) des virtuellen Kanals entspricht, umfasst,

und Mittel (33b) zur Rekonstruktion eines vollständigen Headers (22) ausgehend von einem reduzierten Header (25) durch Einfügung von 4 bzw. 8 Nullen, um die höherwertigen Bits zu bilden.

RESEAU ATM — 11

TERMINAL

```
        ┌──────────────────┐
        │    RECEPTION     │  13
        │  D'UNE CELLULE   │
        └──────────────────┘
               │
        ┌──────────────────┐
        │    REDUCTION     │  14
        │   DE L'ENTETE    │
        └──────────────────┘
               │
        ┌──────────────────┐
        │     CALCUL       │  15
        │    DU  HEC       │
        └──────────────────┘
               │
        ┌──────────────────┐
        │   TRANSMISSION   │  16
        │  CELLULE REDUITE │
        └──────────────────┘
               │
        ┌──────────────────┐
        │   VERIFICATION   │  17
        │    DU  HEC       │
        └──────────────────┘
               │
        ┌──────────────────┐
        │  RECONSTRUCTION  │  18
        │  ENTETE  COMPLET │
        └──────────────────┘
               │
        ┌──────────────────┐
        │   CALCUL DU      │  19
        │  NOUVEAU  HEC    │
        └──────────────────┘
               │
          TERMINAL — 12
```

```
        ┌──────────────────┐
        │   RECEPTION DE   │  110
        │   DONNEES A      │
        │   TRANSMETTRE    │
        └──────────────────┘
               │
        ┌──────────────────┐
        │  CONSTRUCTION DE │  111
        │ L'ENTETE REDUIT  │
        └──────────────────┘
               │
        ┌──────────────────┐
        │     CALCUL       │  112
        │    DU  HEC       │
        └──────────────────┘
               │
        ┌──────────────────┐
        │   TRANSMISSION   │  113
        │  CELLULE REDUITE │
        └──────────────────┘
               │
        ┌──────────────────┐
        │   VERIFICATION   │  114
        │    DU  HEC       │
        └──────────────────┘
               │
        ┌──────────────────┐
        │  RECONSTRUCTION  │  115
        │  ENTETE  COMPLET │
        └──────────────────┘
               │
        ┌──────────────────┐
        │   CALCUL DU      │  116
        │  NOUVEAU  HEC    │
        └──────────────────┘
               │
        ┌──────────────────┐
        │   TRANSMISSION   │  117
        └──────────────────┘
               │
         RESEAU  ATM — 11
```

Fig. 1

Fig. 2

EP 0 987 917 B1

Fig. 3

Fig. 4